Europäisches Patentamt

European Patent Office

Office européen des brevets

(19)

(11) EP 0 911 053 A2

(12) EUROPEAN PATENT APPLICATION

(43) Date of publication:
    28.04.1999  Bulletin 1999/17

(51) Int Cl.⁶: **A61M 16/18**

(21) Application number: **98307972.4**

(22) Date of filing: **30.09.1998**

(84) Designated Contracting States:
    **AT BE CH CY DE DK ES FI FR GB GR IE IT LI LU MC NL PT SE**
    Designated Extension States:
    **AL LT LV MK RO SI**

(30) Priority: **15.10.1997 US 951035**

(71) Applicant: **OHMEDA INC.**
    **Liberty Corner, New Jersey 07938-0804 (US)**

(72) Inventors:
    • **Gorski, Thomas**
      **Belleville, Wisconsin 53508 (US)**
    • **Schoeberle, Thomas**
      **Madison, Wisconsin 53719 (US)**

(74) Representative: **Charlton, Peter John et al**
    **Elkington and Fife**
    **Prospect House**
    **8 Pembroke Road**
    **Sevenoaks, Kent TN13 1XR (GB)**

(54)    **Anesthetic agent liquid metering device**

(57)    An anesthesia system where the flow rate of the fresh gas flow to the patient breathing circuit is monitored. The vaporizer that supplies the anesthetic vapor to the fresh gas stream includes a liquid metering pump that pumps the liquid anesthetic into an evaporating chamber where it is combined with the carrier gas to be introduced into the patient breathing circuit. The liquid metering pump is controlled by the rate of flow of the fresh gas into the evaporating chamber that the concentration of anesthetic vapor in the gases to the patent breathing circuit remains at the desired concentration despite changes in the flow of the fresh gas stream.

FIG. 2

## Description

Background

This invention relates to an anesthesia system for providing anesthesia to a patient undergoing surgery, and, more particularly, to an anesthesia system having improved control of the vaporization of the liquid anesthetic agent.

It is generally necessary for anesthetic agents to be administered to a patient in very accurate dosages during an operation and, to that end, an anesthesia machine is employed that provides the controlled, accurate dosages of such anesthetic.

In general, anesthesia systems are utilized in operating rooms and comprise various equipment necessary to anesthetize the patient and maintain the patient in that state until the operation is completed and it is possible to terminate the introduction of the anesthetic agent.

Such systems comprise various pressure regulators, flow control devices, gas mixing devices and vaporizers to vaporize a volatile liquid anesthetic and to introduce the anesthetic laden gases into the patient. The patient is connected to the system by means of a face mask or other device and which interfaces with the anesthesia system via a patient circuit that may typically have an inspiratory limb through which the gases are introduced into the patient and an expiratory limb that conveys the exhaled gases from the patient.

In one typical anesthesia system, the overall flow of gases to and from the patient may be in a generally closed circuit, commonly referred to as the circle system, that is, the patient is connected to a substantially closed volume supply of gases and rebreathes certain of those exhaled gases supplemented by fresh gas.

As the driving force to the circle breathing circuit, and, of course, to the patient, a ventilator is used and which basically breathes for the patient since the patient is under anesthesia and is unable to carry out the normal spontaneous breathing functions. The ventilator, therefore, provides a quantity of the gas containing a predetermined metered quantity of the anesthetic agent along with other gases such as nitrous oxide and, of course, a life sustaining percentage of oxygen.

That gas containing the anesthetic may typically be delivered through an intermediate mechanism such as a bellows. In such case, the driving gas from the ventilator does not contain the anesthetic agent but is used to simply power the bellows to collapse that bellows to deliver the aforementioned anesthetic containing gas from the bellows to the patient. Instead of drive gas, other driving means such as an electromechanical or mechanical means are also used.

In any of the aforedescribed systems, the anesthetic laden gas is delivered to the inspiratory limb of the circle patient breathing circuit and is introduced into the patient to provide anesthesia to that patient. That anesthetic gas to the inspiratory limb is provided by a source of gases, including fresh gas, oxygen and generally nitrous oxide, that is mixed to a predetermined mixture in a gas mixer and the mixed gases are then passed through an agent vaporizer where the anesthetic agent is introduced into those gases.

In the expiratory limb of the circle patient breathing circuit, as the patient exhales, the exhalation gases pass through the expiratory limb where they are recirculated back to the inspiratory limb where they are again inhaled by the patient. In this manner, the system is closed and which allows the optimum use of the rather expensive anesthetic agent. If the fresh gas added to the circuit exceeds the net of gases taken up by the patient or leaked from the circuit, the excess gases are popped off via a pop-off valve.

Accordingly, the fresh gas for the machine is controlled to provide the correct amount of gas added to the system, however, that flow of fresh gas may vary during the operation for a number of reasons. With most prior art vaporizers, the principle of operation is to have a main stream of fresh gas and a side stream of gas is diverted from the main stream. The side stream passes through the anesthetic agent and becomes saturated with the vapor and then returns to the main stream to achieve the particular concentration desired of anesthetic vapor in the stream of the gas to the patient breathing circuit. A typical example of such a system is shown and described in U.S. Patent 5,146,915 of Montgomery.

At the present, it is becoming more common to use an electronic vaporizer where a metered amount of liquid anesthetic agent is delivered to a vaporizing chamber where it is quickly converted to vapor. In such cases, the flow of liquid controls the amount of vapor that is produced by the vaporizer. Typical examples of such vaporizers are shown and described in U.S. Patent 5,509,405 and U.S. Patent. 5,645,052.

In such electronic vaporizers, the flow of liquid anesthetic is metered by a accurate pump into an evaporating chamber where it is converted into vapor and combined with a carrier gas to produce the gas having a precise concentration of anesthetic vapor to be administered to a patient. The liquid metering pump may be powered by various motive means and a preferred motive means is a stepper motor that controls the metering pump so that the pump supplies a precise amount of liquid to the evaporating chamber.

Accordingly, since there is no direct correlation between the flow of fresh gas into the anesthesia system and the amount of vapor formed in the vaporizer, it would be advantageous to provide that control, that is, to have some control of the vapor created in the evaporating chamber depending upon the flow of fresh gas being provided to the anesthesia system. In that way, a corresponding change can be effected in the amount of vapor produced and combined with the carrier gas accordance with a change in the flow of the fresh gas to the anesthesia machine.

## Summary of the Invention

In accordance with the present invention, an anesthetic vaporizer is provided that in includes a metering pump that meters a precise amount of liquid anesthetic agent into an evaporating chamber and where the flow of fresh gas to the anesthesia machine is monitored in real time. Thus, where the flow of fresh gas changes in accordance with the desired amount of fresh gas, either determined by the clinician or by the automatic control by the anesthesia machine itself, the system of the present invention changes the flow of liquid anesthetic agent into the evaporating chamber in response to and paralleling that change in flow of the fresh gas.

Therefore, with the present system, the vapor added to the fresh gas is dependent upon the flow of that fresh gas and the concentration set by the user, or the machine. There is thus better control of the vapor and the concentration of vapor does not vary when the fresh gas varies, that is, the concentration is maintained at the set value despite the normal changes in the flow of the fresh gas to the system.

## Brief Description of the Drawings

FIG. 1 is a block diagram of the components of an anesthesia system normally used in providing anesthesia to a patient, and

FIG. 2 is a schematic view of the vaporizing system used with the present invention and showing the vaporizer controlled by the fresh gas flow.

## Detailed Description of the Invention

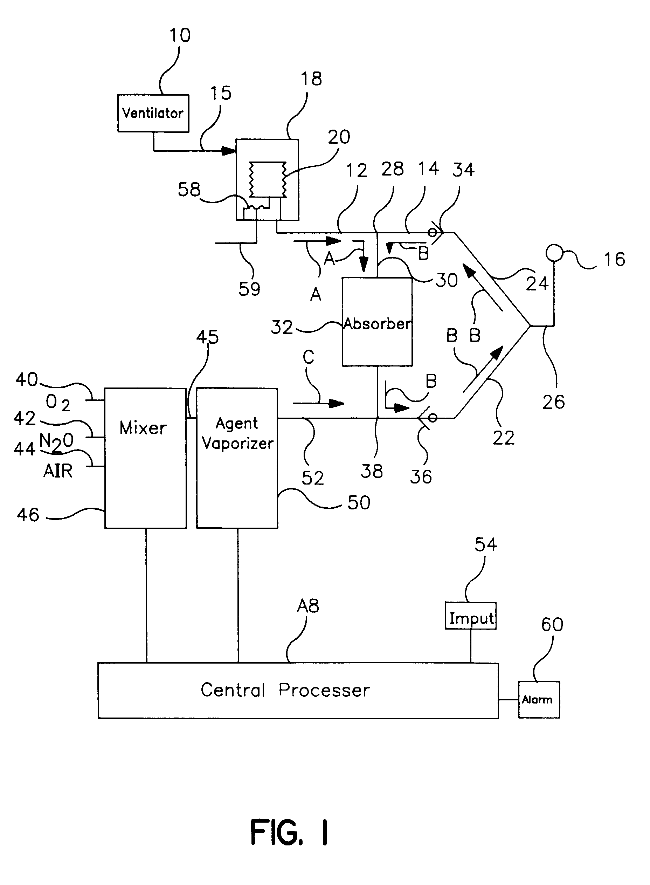

Referring now to FIG. 1, there is shown a block diagram of a typical anesthesia system for providing anesthesia to a patient. As shown, a ventilator 10 is provided and which may be of the type shown and described in U.S. Patent 5,315,989 assigned to the present applicant and the disclosure of which is incorporated herein by reference. The ventilator of the aforementioned U.S. Patent has an inhalation cycle and an exhalation cycle controlled by a central processing unit.

The ventilator 10 provides gas to the patient during the inhalation cycle via a conduit 12 to the patient breathing circuit 14 where it is delivered to the patient 16. The ventilator 10 typically includes a bellows container 18 and air or other powering gas is supplied to the bellows container 18 via conduit 15, exterior of the bellows 20 and which then collapses the bellows 20 to force gas within the bellows 20 to the patient 16.

As also noted in the aforementioned U.S. Patent 5,315,989, the patient breathing circuit 14 itself conventionally includes an inspiratory limb 22 and an expiratory limb 24 and the patient 16 is connected to a wye connection 26 located intermediate the inspiratory and the expiratory limbs 22,24. The means of connection to the patient 16 may be an endotracheal tube, face mask or other interface between the patient 16 and the patient breathing circuit 14.

In conventional operation, gas is delivered to the patient 16 by means of a powering gas from ventilator 10 that collapses the bellows 20 to drive the gas into conduit 12 and then into the tee 28 where the gas enters a conduit 30 and passes through an absorber 32. After passing throuah the absorber 32, the aas enters the inspiratory limb 22 of the patient breathing circuit 14 to be administered to the patient 16. As the patient exhales, that exhalation, now laden with $CO_2$, passes through the expiratory limb 24 where it again passes through the tee 28 and continues to the absorber 32 where the $CO_2$ is eliminated by a $CO_2$ absorbing material, such as sodalime.

A pair of check valves 34 and 36 are positioned in the patient breathing circuit 14 in the expiratory and inspiratory limbs 24 and 22, respectively, to maintain the flow of gas in the proper direction around the circle patient breathing circuit 14.

A flow of fresh gas is also introduced into the patient breathing circuit 14 and, as shown, is added at a tee 38 and thus into the inspiratory limb 22 of the patient breathing circuit 14. That flow of fresh gas is provided from a source of gas, typically oxygen, air and nitrous oxide to aid in anesthetizing the patient. As shown in FIG. 1, there is a supply of oxygen 40, nitrous oxide 42 and air 44 and such supplies may be through a central piping system of a hospital or may be through the use of individual cylinders of such gases. A flow sensor 45 is provided in the fresh gas line between the gas mixer 46 and the agent vaporizer as will be later explained and is used to monitor the flow of fresh gas in the provided to the patient breathing circuit 14.

In any event, the gases are mixed in a gas mixer 46 in the proportion desired by the user. The actual control of the proportions and the flow through the gas mixer 46 is, in the preferred embodiment, controlled by a central processing unit (CPU) 48 as described in the aforementioned U.S. Patent. The mixed gas from the gas mixer 46 then passes through the agent vaporizer 50 where liquid anesthetic agent is vaporized and added to the stream of gas such that anesthetic laden gas continues into a conduit 52 and enters the patient breathing circuit 14 at the tee 38.

Again, in the preferred embodiment, the control of the agent vaporizer 50 is by means of the CPU 48 and which determines the percentage concentration of anesthetic agent that is in the gas that enters the patient breathing circuit 14 and thus that is supplied to the patient 16 to induce and maintain anesthesia.

The CPU 48 is, in turn, controlled by an input device 54 provided so that the clinician can input the data needed to determine the various parameters to provide the fresh gas flow and anesthetic concentration desired to anesthetize the patient. The input to the CPU 48 may be the flow of fresh gas to the patient as well as the concentrations of the various gases mixed in the gas mixer 46 and the concentration of anesthetic to be added to the mixed fresh gas by the agent vaporizer 50.

The overall flow scheme of the present conventional system is therefore such that the gas in the bellows 20 is forced by the ventilator 10 into conduit 12 in accordance with the arrows A during the inhalation cycle of the patient 16. The gas thus passes through the tee 28 and through absorber 32 where it further passes through tee 38 and into the inspiratory limb 22 of the patient breathing circuit 14. At tee 38, fresh gas containing a predetermined concentration of an anesthetic agent is joined with the gas from the bellows 20 and proceeds with the gases already circulating in patient breathing circuit 14 and administered to the patient 16.

When the patient exhales, the exhaled gas passes through the expiratory limb 24 of the patient breathing circuit 14 through tee 28 and continues through the conduit 12 and into the bellows 20. At the same time, fresh gas that continuously flows into the circuit 14 from conduit 52 is also directed towards the bellows 20 after passing through the patient breathing circuit 14. When the bellows 20 reaches the end of its travel, any excess gas is popped off from the bellows 20 via pop-off valve 58 and exits the system via conduit 59 and typically into a gas scavenging system, not shown.

During the inspiratory phase, the bellows 20 is driven downwardly by the ventilator 10. The unidirectional check valves 34 and 36 direct the gas from the bellows 20 to conduit 12 and through the absorber 32 where the gas is scrubbed of $CO_2$. Also directed is the fresh gas from conduit 52 towards the patient 16 via inspiratory limb 22 of breathing circuit 14.

As can be seen, therefore, the anesthesia system is basically a circle system where the gas continues to pass in a circle as shown by the arrows B with the addition of fresh gas and the anesthetic agent added to that gas in the direction of Arrow C as the gas passes around the circle.

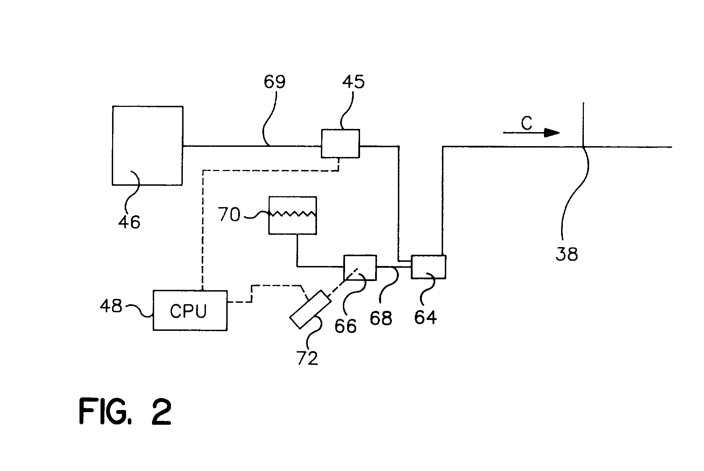

Turning now to FIG. 2, there is shown a schematic view of the vaporizing scheme for the control of the vapor delivered to the fresh gas for the anesthesia patient breathing circuit 14. As shown, the gas mixer 46 provides the flow of carrier gas to an evaporating chamber 64 where evaporation of liquid anesthetic takes place. Liquid is provided to the evaporating chamber 64 by means of a liquid metering pump 66 and which supplies that liquid through a conduit 68 and into a suitable means to carry out the evaporation of the liquid in that evaporating chamber 64. In the preferred embodiment, the liquid may be sprayed into a heated evaporating chamber so that the liquid is quickly evaporated and combined with the carrier gas from the gas mixer 46 via a conduit 69. The combined carrier gas and vapor passes into the conduit 52 and on to the tee 38 to be delivered to the patient.

As can be seen, the CPU 48 controls the liquid metering pump 66 to control the flow of liquid anesthetic agent into the evaporating chamber 64 to be converted into vapor. The liquid metering pump 66, in turn, receives the supply of liquid anesthetic from a reservoir 70.

By controlling the speed of the liquid metering pump 66, therefore, one can control the amount of liquid to be evaporated in the evaporating chamber 64 and that liquid metering pump 66 may be operated by a motive means such as a stepper motor 72. Accordingly, by controlling the sped of the stepper motor 72, the flow of liquid into the evaporating chamber 64 is controlled by the CPU 48.

The flow sensor 45 thus monitors the flow of the fresh gas from the gas mixer 46 on a real time basis, that is, taking samples at the rate of about 1000 times per second. That flow rate information is transmitted to the CPU 48 where it is converted into a signal to the stepper motor 72 so that the rotation of the stepper motor 72 is directly controlled by the flow of carrier gas from the gas mixer 46 into the evaporating chamber 64.

As shown, the stepper motor 72 is the preferred motive means as having good control of the rotation, however, other metering pumps can be used and controlled by other motive means providing the accuracy of control is present.

In determining the amount of liquid per flow of the fresh gas, a simple derivation can be made by means of the equation for the vapor flow rate

$$\text{Vapor (L/sec.)} = \text{Fresh Gas (L/sec.)} * [C/(100-C)]$$

Where C is the concentration of drug vapor in % (vol/vol)

By using a known scaling constant which is derived as a ration of the densities of the liquid phase and the vapor phase ( the constant $K_R$ is approximately 200 for most anesthetics), we have

$$\text{Liquid (uL/sec.)} = \text{Fresh Gas (L/sec.)} * K_R * [C/(100-C)]$$

and which provides the amount of liquid per second for the flow of the fresh gas. Accordingly with the normal changes in the fresh gas flow during anesthesia, the present invention can maintain the concentration at the desired value even when the changes in fresh gas flow take place.

Numerous further variations and combinations of the features discussed above can be utilized without departing from the spirit of the invention as defined by the claims below. Accordingly, the foregoing description of the preferred embodiment should be taken by way of illustration rather than by way of limitation of the invention as claimed.

## Claims

1. An anesthesia system for delivering a carrier gas laden with an anesthetic vapor to a patient, said system having a patient breathing circuit connected to a patient for administering the carrier gas laden with anesthetic vapor to a patient, a source of a carrier gas, a source of a liquid anesthetic agent, an evaporating chamber, a first conduit for communicating said liquid anesthetic agent from said source of liquid anesthetic agent to said evaporating chamber, control means to control the flow of said liquid anesthetic agent through said first conduit, a second conduit for communicating carrier gas from said source of carrier gas to said evaporating chamber, said evaporating chamber evaporating the liquid anesthetic agent to mix with the carrier gas and produce the carrier gas laden with anesthetic vapor to said patient breathing circuit, means to determine the flow of carrier gas introduced into said evaporating chamber, and means to control the flow of liquid anesthetic agent through said first conduit at a rate dependent on the flow of the carrier gas in said second conduit.

2. An anesthesia system as defined in Claim 1 wherein said means to determine the flow of carrier gas into said evaporating chamber comprises a flow sensor in said second conduit.

3. An anesthesia system as defined in Claim 1 wherein said control means to control the flow of liquid anesthetic agent in said first conduit comprises a variable speed pump.

4. An anesthesia system as defined in Claim 3 wherein said variable speed pump is rotated by a stepper motor.

5. An anesthesia system as defined in Claim 2 wherein said flow sensor senses the flow at a rate of about 1000 times per second.

6. An anesthesia system for delivering a carrier gas laden with an anesthetic vapor to a patient, said system having a patient breathing circuit connected to a patient for administering the carrier gas laden with anesthetic vapor to a patient, a source of a carrier gas, a source of a liquid anesthetic agent, a vaporizer comprising a liquid metering pump receiving liquid anesthetic agent from said source of liquid anesthetic agent and having an evaporating chamber for receiving liquid from said metering pump at a controllable rate and for receiving the flow of carrier gas, said evaporating chamber evaporating the liquid to provide the carrier gas laden with anesthetic vapor to said patient breathing circuit, means to determine the flow of carrier gas introduced into said evaporating chamber, and means to control said metering pump to pump said liquid anesthetic into said evaporating chamber at a rate dependent on the flow of the carrier gas monitored by said sensor.

7. An anesthesia system as defined in Claim 6 wherein said liquid metering pump comprises a positive displacement pump.

8. An anesthesia system as defined in Claim 7 wherein said positive displacement pump is rotated by a variable speed motor.

9. An anesthesia system as defined in Claim 8 wherein said variable speed motor is a stepper motor.

10. An anesthesia system as defined in Claim 5 wherein said means to determine the flow of carrier gas to said evaporating chamber comprises a flow sensor sensing the flow of carrier gas from said carrier gas source.

11. A method of controlling the evaporation of a liquid anesthetic to produce a flow of carrier gas laden with the anesthetic vapor comprising the steps of:

    providing an evaporating chamber for evaporating a liquid anesthetic;

    introducing a flow of a carrier gas into the evaporating chamber;

    introducing a flow of liquid anesthetic agent into the evaporating chamber at a known rate,

    evaporating the liquid within the evaporating chamber to mix with the carrier gas to produce a carrier gas laden with anesthetic agent vapor, and

    controlling the flow of the liquid anesthetic into the evaporating chamber based upon the flow of carrier gas into the evaporating chamber.

FIG. I

FIG. 2

EP 0 911 053 A2